# EUROPÄISCHE PATENTSCHRIFT

(11) **EP 3 248 173 B1**
(45) Veröffentlichungstag und Bekanntmachung des Hinweises auf die Patenterteilung: **06.11.2019**
(21) Anmeldenummer: 16703924.7
(22) Anmeldetag: 21.01.2016
(51) Int. Cl.: G06T 7/12, G06T 7/136, G06T 7/73, G06K 9/32, G06K 9/46

(54) **ASSISTENZEINRICHTUNG UND VERFAHREN ZUR BILDGEBENDEN UNTERSTÜTZUNG EINES OPERATEURS WÄHREND EINES CHIRURGISCHEN EINGRIFFS UNTER VERWENDUNG MINDESTENS EINES MEDIZINISCHEN INSTRUMENTES**
ASSISTANCE DEVICE AND METHOD FOR IMAGING SUPPORT OF AN OPERATING SURGEON DURING A SURGICAL PROCEDURE USING AT LEAST ONE MEDICAL INSTRUMENT
DISPOSITIF D'ASSISTANCE ET PROCÉDÉ D'AIDE PAR IMAGERIE D'UN CHIRURGIEN PENDANT UNE INTERVENTION CHIRURGICALE UTILISANT AU MOINS UN INSTRUMENT MEDICAL

(30) Priorität: 22.01.2015 DE 102015100927
(43) Veröffentlichungstag der Anmeldung: 29.11.2017
(73) Patentinhaber: Maquet GmbH, 76437 Rastatt (DE)
(72) Erfinder: GAUVIN, Aurélien, 18330 Nancy (FR); BAUMUNG, Peter, 76137 Karlsruhe (DE); IBACH, Bastian, 76185 Karlsruhe (DE); FRANZ, Thilo, 76137 Karlsruhe (DE); SIMONOVSKY, Martin, 69115 Heidelberg (DE)
(74) Vertreter: Zacco GmbH
(86) Internationale Anmeldenummer: PCT/EP2016/051189
(87) Internationale Veröffentlichungsnummer: WO 2016/116537

(56) Entgegenhaltungen:
- DARRIN R. UECKER ET AL: "Automated instrument tracking in robotically assisted laparoscopic surgery", JOURNAL OF IMAGE GUIDED SURGERY, Bd. 1, Nr. 6, 1. Januar 1995 (1995-01-01), Seiten 308-325, XP055269122, US ISSN: 1078-7844, DOI: 10.1002/(SICI)1522-712X(1995)1:6<308::AID- IGS3>3.0.CO;2-E
- DOIGNON C ET AL: "Detection of grey regions in color images : application to the segmentation of a surgical instrument in robotized laparoscopy", INTELLIGENT ROBOTS AND SYSTEMS, 2004. (IROS 2004). PROCEEDINGS. 2004 I EEE/RSJ INTERNATIONAL CONFERENCE ON SENDAI, JAPAN 28 SEPT.-2 OCT., 2004, PISCATAWAY, NJ, USA,IEEE, PISCATAWAY, NJ, USA, Bd. 4, 28. September 2004 (2004-09-28), Seiten 3394-3399, XP010766215, DOI: 10.1109/IROS.2004.1389941 ISBN: 978-0-7803-8463-7
- REDDI S S ET AL: "An optimal multiple threshold scheme for image segmentation", IEEE TRANSACTIONS ON SYSTEMS, MAN AND CYBERNETICS, IEEE INC. NEW YORK, US, Bd. SMC-14, Nr. 4, 1. Juli 1984 (1984-07-01), Seiten 661-665, XP011464970, ISSN: 0018-9472, DOI: 10.1109/TSMC.1984.6313341

## Beschreibung

Die Erfindung betrifft eine Assistenzeinrichtung zur bildgebenden Unterstützung eines Operateurs während eines chirurgischen Eingriffs unter Verwendung mindestens eines medizinischen Instrumentes, mit einer Kamera zum Erzeugen eines Videosignals, das eine Bildsequenz von Einzelbildern beinhaltet, einem Sichtgerät zum Darstellen der Bildsequenz auf Grundlage des Videosignals, einer Bilderverarbeitungseinheit mit einem Instrumentenerfassungsmodul zum Erfassen mindestens einer das verwendete Instrument in dem jeweiligen Einzelbild repräsentierenden Zielstruktur durch Identifizieren eines vorbestimmten Erkennungsmerkmals und zum Extrahieren einer Positionsinformation, welche die Lage der Zielstruktur in dem Einzelbild angibt, einem mit der Kamera gekoppelten Manipulator, der zum Bewegen der Kamera über ein Steuersignal steuerbar ist, und einer Manipulatorsteuerung zum Erzeugen eines Steuersignals aus der Positionsinformation und zum Ansteuern des Manipulators über das Steuersignal.

Aus dem Stand der Technik sind Assistenzeinrichtungen bekannt, die einen Operateur während eines chirurgischen Eingriffs wirksam unterstützen. So betrachtet der Operateur beispielsweise während eines laparoskopischen Eingriffs den Operationssitus indirekt über ein Sichtgerät, z.B. einen Monitor, auf dem die laparoskopischen Instrumente zu sehen sind, mit denen er die anatomische Struktur im Patienten manipuliert. Das auf dem Monitor in Echtzeit angezeigte Videobild wird von einer Kamera aufgenommen, die Teil eines Endoskops ist und auf den Operationssitus gerichtet ist.

Während des Eingriffs sollte die Kamera so auf den Operationssitus gerichtet sein, dass sich der behandelte Zielbereich mit den Instrumentenspitzen und der zu manipulierenden anatomischen Struktur etwa in der Mitte des auf dem Sichtgerät dargestellten Videobildes befindet. Soll der betrachtete Bildausschnitt verändert werden, so muss die Kamera im Körper des Patienten bewegt werden. Dabei sollte der Zielbereich wieder in der Mitte des Videobildes angezeigt werden.

Um die Endoskopkamera in der gewünschten Weise nachzuführen, ist sie mit einem Manipulator, z.B. einem Roboterarm, gekoppelt, der entsprechend der gewünschten Kamerabewegung verstellt wird. Hierzu wird er von einer Manipulatorsteuerung über ein Steuersignal, das aus der Position der in dem Videobild erfassten Instrumentenspitze gewonnen wird, angesteuert. Eine Assistenzeinrichtung vorstehend beschriebener Art ist beispielsweise in der DE 199 61 971 B4 beschrieben.

Herkömmliche Assistenzeinrichtungen arbeiten üblicherweise mit Markierungen, die an der Instrumentenspitze angebracht werden und so ein Erkennungsmerkmal bilden, das die Erfassung der Instrumentenspitze in dem Videobild ermöglicht. Anstelle einer solchen Markierung können auch inhärente Eigenschaften des gerade verwendeten medizinischen Instrumentes, wie dessen spezielle Form oder spezielle Farbe als Erkennungsmerkmal genutzt werden. Nachteilig an diesen Lösungen ist jedoch, dass das zu identifizierende Erkennungsmerkmal eine spezielle Eigenschaft des gerade verwendeten Instrumentes repräsentiert. Eine Erkennung beliebiger Instrumente ist so nicht möglich.

Der Artikel "Automated Instrument Tracking in Robotically-Assisted Laparoscopic Surgery" von Darrin R. Uecker et al., veröffentlicht im Journal of Image Guided Surgery, Bd. 1, Nr. 6, Seiten 308-325, beschreibt einen Algorithmus zur Bildanalyse und zum Nachverfolgen, um bei Roboter-gestützten laparoskopischen Eingriffen eine automatisierte Instrumentenortung zu erreichen.

Der Artikel "Detection of grey regions in color images: application to the segmentation of a surgical instrument in robotized laparoscopy" von Christophe Doignon et al., veröffentlicht auf Seiten 3394 bis 3399 des vierten Tagungsbands der internationalen Konferenz über intelligente Roboter und Systeme, die vom 28. September bis 2. Oktober 2004 in Sendai in Japan stattfand, beschreibt die Erkennung und Positionsbestimmung von Graubereichen in Farbbildern.

Der Artikel "An Optimal Multiple Threshold Scheme for Image Segmentation" von S. S. Reddi et al., veröffentlicht in den IEEE Transactions on Systems, Man, and Cybernetics, Vol. SMC-14, Nr. 4, Juli/August 1984, Seiten 661 bis 665, beschreibt ein Verfahren zum Bestimmen einzelner oder mehrerer Schwellwerte, welche die Interklassenvarianz zwischen dunklen und hellen Bereichen maximieren.

Aufgabe der Erfindung ist es, eine Assistenzeinrichtung anzugeben, die eine weitgehend selbsttätige Instrumentenerkennung insbesondere ohne Verwendung eigens hierfür vorgesehener Markierungen ermöglicht.

Die Erfindung löst diese Aufgabe bei einer Assistenzeinrichtung eingangs beschriebener Art durch die kennzeichnenden Merkmale des Anspruchs 1.

Die erfindungsgemäße Lösung beruht zum einen auf der Erkenntnis, dass die auf dem vorliegenden technischen Gebiet üblicherweise verwendeten medizinischen Instrumente aus weitgehend achromatischen Materialien gefertigt sind, d.h. eine Farbsättigung aufweisen, die deutlich kleiner als die Farbsättigung der in dem zu behandelnden Zielbereich befindlichen anatomischen Struktur ist. Zum zweiten beruht die Erfindung auf der Erkenntnis, dass nahezu jedes verwendbare Instrument in weiten Teilen ein gerades und starres Objekt bildet. Vor diesem Hintergrund sieht es die erfindungsgemäße Lösung vor, das von dem Instrumentenerfassungsmodul zu identifizierende Erkennungsmerkmal als ein Bildsegment zu identifizieren, das zum einen eine vergleichsweise geringe Farbsättigung hat und zum anderen eine wenigstens zum Teil geradlinig verlaufende Umrisslinie aufweist. Anhand dieses Erkennungsmerkmals ist es möglich, nahezu jedes Instrument in dem Videobild gegenüber der im Zielbereich vorhandenen anatomischen Struktur abzugrenzen. Insbesondere ist dies ohne Verwendung einer an dem Instrument anzubringenden Markierung möglich.

Die als Erkennungsmerkmal zu definierende obere Grenze der Farbsättigung wird so gewählt, dass die bei herkömmlichen medizinischen Instrumenten messbaren Farbsättigungen zuverlässig unterhalb dieser oberen Grenze liegen. Wie schon oben erwähnt, ist dies möglich, da herkömmliche Instrumente in der Regel aus Materialien gefertigt sind, die weitgehend achromatisch sind und deshalb eine weitaus geringere Farbsättigung als die umliegenden anatomische Struktur im Zielgebiet aufweisen.

Die Bildverarbeitungseinheit enthält ein Segmentierungsmodul, das auf Grundlage des jeweiligen Grauwertbildes mehrere Binärbilder erzeugt, in denen das Instrumentenerfassungsmodul das Bildsegment identifiziert. Die Bildpunkte dieses Binärbildes nehmen einen von zwei möglichen Binärwerten an, je nachdem, ob die ihnen entsprechenden Bildpunkte des Einzelbildes eine dem vordefinierten Erkennungsmerkmal entsprechende Farbsättigung aufweisen oder nicht. Beispielsweise kann auf diese Weise ein Schwarz-Weiß-Binärbild erzeugt werden, dessen weiße Bildpunkte oder Pixel eines oder mehrere Instrumente repräsentieren und dessen schwarze Bildpunkte die anatomische Struktur darstellen.

Die Bildverarbeitungseinheit enthält ein Vorverarbeitungsmodul, das auf Grundlage des jeweiligen Einzelbildes ein Grauwertbild erzeugt, dessen Bildpunkten jeweils ein Grauwert zugeordnet ist, der die Farbsättigung des entsprechenden Bildpunktes des Einzelbildes repräsentiert. Das Segmentierungsmodul erzeugt dann auf Grundlage des Grauwertbildes die Binärbilder, deren Binärbildpunkten ein erster Binärwert zugeordnet ist, wenn die zugehörigen Grauwerte gleich oder kleiner als ein der vordefinierten Farbsättigung entsprechender Schwellwert ist, und deren Binärbildpunkten ein zweiter Binärwert zugeordnet ist, wenn die zugehörigen Grauwerte größer als der Schwellwert sind. Das erzeugte Grauwertbild stellt somit ein Farbsättigungsbild dar, aus dem sich ohne großen technischen Aufwand mehrere Binärbilder ableiten lassen, die der weiteren Bildverarbeitung zugrunde zu legen sind.

Das Instrumentenerfassungsmodul fasst mehrere linear voneinander beabstandete geradlinige Abschnitte der Umrisslinie zu einer durchgehenden Kantenlinie zusammen, die eine Kante des Instruments repräsentiert. Dies ist dann von Vorteil, wenn in den Binärbildern, die dem jeweiligen Einzelbild zugeordnet sind, mehrere, einander kreuzende Instrumente dargestellt sind. Da davon auszugehen ist, dass ein jeweiliges Instrument eine gerade Form aufweist, kann durch die Zusammensetzung kollinear beabstandeter Umrissabschnitte das zu diesen Abschnitten gehörende Instrument gleichsam aus der gekreuzten Instrumentenanordnung herausgefiltert werden. Dies erleichtert die Instrumentenerkennung, wenn während des chirurgischen Eingriffs mehrere Instrumente verwendet werden.

Vorzugsweise paart das Instrumentenerfassungsmodul zur Erfassung der Zielstruktur jeweils zwei parallel zueinander angeordnete Kantenlinien miteinander. Diese Kantenpaarung beruht auf der Überlegung, dass bei Erfassung mehrerer parallel zueinander angeordneter Kantenlinien eine hohe Wahrscheinlichkeit dafür besteht, dass diese Kantenlinien zu ein- und demselben Instrument gehören. Dies ermöglicht eine noch zuverlässigere Instrumentenerkennung.

Das Segmentierungsmodul erzeugt auf Grundlage des jeweiligen Grauwertbildes nicht nur ein, sondern gleich mehrere Binärbilder und wendet für deren Erzeugung unterschiedliche Schwellwerte an. Demnach wird für jedes dieser Binärbilder eine eigene obere Grenze für die Farbsättigung als Erkennungsmerkmal vorgegeben. Dies ist insbesondere dann von Vorteil, wenn ein- und dasselbe Instrument aus verschiedenen Materialien besteht, die unterschiedliche Farbsättigungen aufweisen. So kann beispielsweise die Spitze eines solchen Instrumentes aus Metall und der Instrumentenschaft aus Kunststoff gefertigt sein. Entsprechendes gilt, wenn mehrere Instrumente verschiedener Materialien und damit unterschiedlicher Farbsättigungen in dem Einzelbild vorhanden sind. Wird in einem solchen Fall der für die Erzeugung des Binärbildes vorgesehene Schwellwert so festgelegt, dass sich etwa Teile aus Kunststoff gut in dem Binärbild segmentieren lassen, so bedeutet dies noch nicht, dass dies auch mit demselben Schwellwert in gleicher Qualität für Metallteile möglich ist. In diesem Fall ist es günstig, nicht nur ein einziges Binärbild auf Grundlage eines einzigen Schwellwertes zu erzeugen, sondern eine Vielzahl von Binärbildern, der eine entsprechende Vielzahl von Schwellwerten zugrunde gelegt wird. Diese Binärbilder bilden auch unter schwierigen Bedingungen eine zuverlässige Basis für eine präzise Instrumentenerkennung. Außerdem lässt sich die Erzeugung mehrerer Binärbilder gut parallelisieren, was im Hinblick auf die Verarbeitungsgeschwindigkeit von Vorteil ist.

Das Instrumentenerfassungsmodul identifiziert in den auf Grundlage des jeweiligen Einzelbildes erzeugten Binärbildern einander entsprechende geradlinige Abschnitte und fasst diese zu einer einzigen Kantenlinie zusammen, die eine Kante des Instrumentes repräsentiert. Da bei dieser Ausgestaltung die Vielzahl von Binärbildern unter Anwendung unterschiedlicher Schwellwerte erzeugt werden, verschiebt sich eine identifizierte Umrisslinie, die den Übergang zwischen einem die anatomische Struktur darstellenden Bildsegment hoher Farbsättigung und einem das Instrument darstellenden Bildsegment geringer Farbsättigung bildet, gleichsam von Binärbild zu Binärbild. Betrachtet man sich die Binärbilder nun gleichsam in ihrer Überlagerung, so ergibt sich eine räumlich eng beieinander liegende Anordnung nahezu paralleler Umrissabschnitte, aus der sich zuverlässig die Instrumentenkante ableiten lässt.

In einer bevorzugten Ausführung enthält die Bildverarbeitungseinheit ein Nachverfolgungsmodul, das die von dem Instrumentenerfassungsmodul erfasste Zielstruktur über mehrere aufeinanderfolgende Einzelbilder nachverfolgt. Die Manipulatorsteuerung erzeugt das auf das jeweilige Einzelbild bezogene Steuersignal zur Ansteuerung des Manipulators aus der Positionsinformation der Zielstruktur nur dann, wenn diese Zielstruktur von dem Nachverfolgungsmodul schon über mehrere aufeinander folgende Einzelbilder nachverfolgt ist. Zur nachführenden Ansteuerung des Manipulators werden demnach nur Zielstrukturen herangezogen, die sich über mehrere aufeinander folgende Einzelbilder nachverfolgen lassen. Ein vermeintlich erkanntes Instrument, das nur in einem einzigen Einzelbild auftaucht, bleibt so für die Nachführung des Manipulators unberücksichtigt. Dies ermöglicht eine fehlerfreie und gleichmäßige Manipulatornachführung.

Vorzugsweise ordnet das Nachverfolgungsmodul der von dem Instrumentenerfassungsmodul in dem jeweiligen Einzelbild erstmalig erfassten Zielstruktur einen Tracker zu und verfolgt diese in den nachfolgenden Einzelbildern erfasste Zielstruktur anhand des Trackers. Unter einem Tracker ist hierbei ein Datenverarbeitungselement zur Repräsentation eines erkannten Instrumentes zu verstehen.

Bei einer besonders bevorzugten Ausführung enthält die Bildverarbeitungseinheit ein Flussmodul, das einen optischen Fluss der Bildsequenz erfasst, der eine in der Bildsequenz enthaltene Bewegungsinformation repräsentiert. Anhand des erfassten optischen Flusses lässt sich vorhersagen, in welche Richtung und mit welcher Geschwindigkeit sich der dem erkannten Instrument zugeordnete Tracker in den folgenden Einzelbildern bewegt.

Vorzugsweise umfasst das Nachverfolgungsmodul ein erstes Teilmodul, das dem Instrumentenerfassungsmodul in Bildverarbeitungsrichtung vorgeordnet ist, und ein zweites Teilmodul, das dem Instrumentenerfassungsmodul in Bildverarbeitungsrichtung nachgeordnet ist. Das erste Teilmodul trifft unter Berücksichtigung des in dem Flussmodul erfassten optischen Flusses für das nächste Einzelbild, das von dem Instrumentenerfassungsmodul noch nicht verarbeitet worden ist, eine Vorhersage über die Positionsinformation des Trackers. Das zweite Teilmodul überprüft für dieses nächste Einzelbild, das unterdessen von dem Instrumentenerfassungsmodul verarbeitet worden ist, die von dem ersten Teilmodul getroffene Vorhersage anhand der von dem Instrumentenerfassungsmodul erfassten Positionsinformation des Trackers. Dadurch wird eine besonders zuverlässige Nachverfolgung des in der Bildsequenz erkannten Instrumentes möglich.

Das Vorverarbeitungsmodul ist vorzugsweise ausgebildet, an dem jeweiligen Einzelbild einen Weißabgleich vorzunehmen. Dieser Weißabgleich wirkt möglichen Fehlkalibrierungen entgegen, die eine Instrumentenerkennung auf Grundlage der chromatischen Eigenschaften des Instrumentes, insbesondere der Farbsättigung, beeinträchtigen könnten.

In einer bevorzugten Ausführung enthält die Bildverarbeitungseinheit ein Parameteroptimierungsmodul, das die Einzelbilder asynchron zu dem Vorverarbeitungsmodul verarbeitet und daraus eine Ansteuerinformation generiert, die dem Vorverarbeitungsmodul vorgibt, ob es einen Weißabgleich vorzunehmen hat oder nicht. Durch die asynchrone Arbeitsweise des Parameteroptimierungsmoduls kann die Überprüfung, ob ein Weißabgleich notwendig ist oder nicht, als ein zu der übrigen Bildverarbeitung nebenläufiger Prozess realisiert werden. So ist es beispielsweise möglich, die Überprüfung gleichsam stichprobenartig innerhalb der Bildsequenz vorzunehmen, etwa nur jedes n-te Einzelbild zu überprüfen, wobei n eine natürliche Zahl größer 1 ist.

Das Parameteroptimierungsmodul kann auf Grundlage der asynchronen Verarbeitung der Einzelbilder auch die Schwellwerte für das Segmentierungsmodul vordefinieren. Dies ist beispielsweise dann von Vorteil, wenn während des chirurgischen Eingriffs Instrumente ausgetauscht werden und sich damit deren Farbsättigungen innerhalb der Bildsequenz instantan drastisch andern. Eine Anpassung der den Farbsättigungen entsprechenden Schwellwerte mit Hilfe des asynchron arbeitenden Parameteroptimierungsmoduls ist hier eine geeignete Maßnahme, auf solche Änderungen zu reagieren.

Die Erfindung betrifft ferner ein Verfahren zur bildgebenden Unterstützung eines Operateurs während eines chirurgischen Eingriffs. Die Merkmale, die vorstehend und im Weiteren in Bezug auf die erfindungsgemäße Assistenzeinrichtung beschrieben sind, sind auch Teil des beanspruchten Verfahrens.

Die Erfindung wird im Folgenden anhand der Figuren näher erläutert. Darin zeigt:
Figur 1: ein Blockdiagramm, das den Gesamtaufbau einer erfindungsgemäßen Assistenzeinrichtung zeigt;
Figur 2: ein Blockdiagramm, das den Aufbau einer Bildverarbeitungseinheit der Assistenzeinrichtung zeigt;
Figur 3: ein Diagramm, das den Ablauf einer in dem erfindungsgemäßen Verfahren durchgeführten Bildvorverarbeitung zeigt;
Figur 4: ein Diagramm, das den Ablauf einer in dem erfindungsgemäßen Verfahren durchgeführten Instrumentenerfassung zeigt;
Figur 5: ein Diagramm, das den Ablauf einer in dem erfindungsgemäßen Verfahren durchgeführten Instrumentennachverfolgung zeigt;
Figur 6: ein in dem erfindungsgemäßen Verfahren erzeugtes Binärbild; und
Figur 7: eine aus dem Binärbild nach Figur 6 gewonnene Umrisslinie.

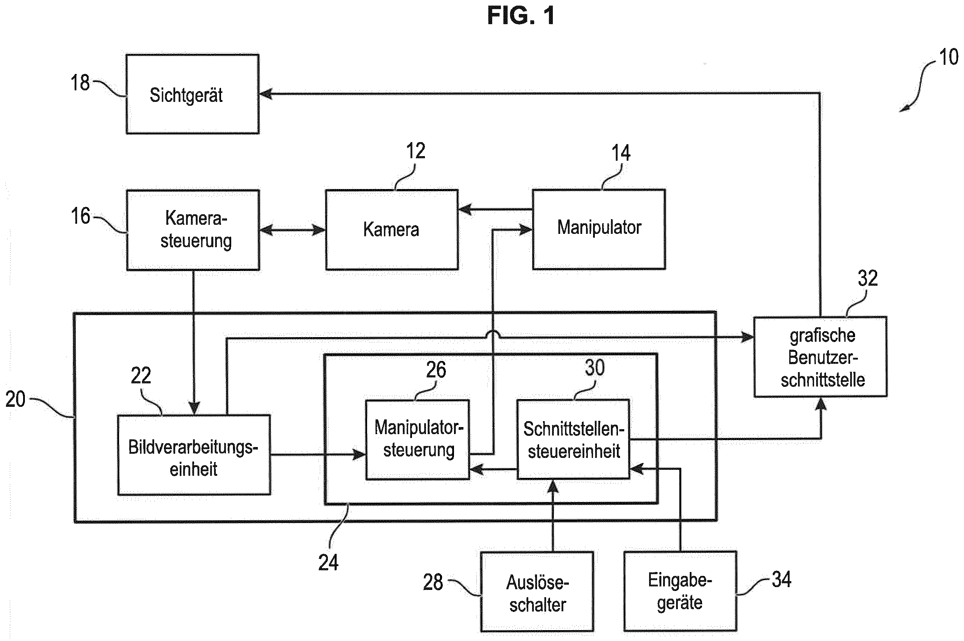

Figur 1 zeigt eine erfindungsgemäße Assistenzeinrichtung 10 in einem Blockdiagramm.

Die Assistenzeinrichtung 10 umfasst eine Kamera 12, die Teil eines nicht explizit gezeigten Endoskops ist, das von einem Manipulator 14, beispielsweise einem Roboterarm, gehalten wird. Der Manipulator 14 weist mechanische Freiheitsgrade auf, die eine Nachführung der Kamera 12 ermöglichen.

Die Kamera 12 fängt ein Videobild eines Zielbereichs innerhalb des menschlichen Körpers auf, in dem sich die zu behandelnde anatomische Struktur befindet. Die Kamera 12 erzeugt demnach ein Videosignal, das eine Bildsequenz von Einzelbildern beinhaltet und in Form eines Datenstroms an eine Kamerasteuerung 16 ausgegeben wird. Die Kamerasteuerung 16 gibt das Videosignal an ein Sichtgerät 18 aus, z.B. einen Monitor, auf dem ein dem Videosignal entsprechendes Videobild der behandelten anatomischen Struktur dargestellt wird.

Die Kamerasteuerung 18 leitet das Videosignal über ein in Figur 1 nicht gezeigtes Bilderfassungsmodul, beispielsweise einen sogenannten Frame-Grabber, einer Steuerung 20 zu. Die Steuerung 20 enthält eine Bildverarbeitungseinheit 22, welches das ihr zugeführte Videosignal als Eingangssignal nutzt, um in später genauer erläuterter Weise eine Instrumentenerkennung durchzuführen. Im Rahmen dieser Instrumentenerkennung werden die in dem Videobild sichtbaren chirurgischen Instrumente von der Bildverarbeitungseinheit 22 erkannt und nachverfolgt. Dabei werden Positionsinformationen gewonnen, die einer Steuereinheit 24 zugeführt werden. Die Steuereinheit 24 enthält eine Manipulatorsteuerung 26. Diese fungiert als Bahnsteuerung, die aus den ihr zugeführten Positionsinformationen ein Steuersignal erzeugt, über das der Manipulator 14 angesteuert wird. Diese Ansteuerung erfolgt beispielsweise derart, dass der Manipulator 14 die an ihm gehaltene Kamera 12 so nachführt, dass die Spitze eines Instrumentes in dem Videobild, das auf dem Sichtgerät 18 angezeigt wird, stets in der Bildmitte angeordnet ist.

Die Assistenzeinrichtung 10 weist ferner einen Auslöseschalter 28 auf, der mit einer in der Steuereinheit 24 enthaltenen Schnittstellensteuereinheit 30 gekoppelt ist. Durch Betätigen des Auslöseschalters 28 wird die Schnittstellensteuereinheit 30 veranlasst, die Manipulatorsteuerung 26 zur Nachführung der Kamera 12 zu aktivieren.

Die Assistenzeinrichtung 10 weist ferner eine graphische Benutzerschnittstelle 32 auf, die einerseits mit der Bildverarbeitungseinheit 22 und der Schnittstellensteuereinheit 30 und andererseits mit dem Sichtgerät 18 gekoppelt ist. Darüber hinaus umfasst die Assistenzeinrichtung 10 weitere Eingabegeräte, die für das Verständnis der vorliegenden Erfindung von untergeordneter Bedeutung und in Figur 1 allgemein mit 34 bezeichnet sind.

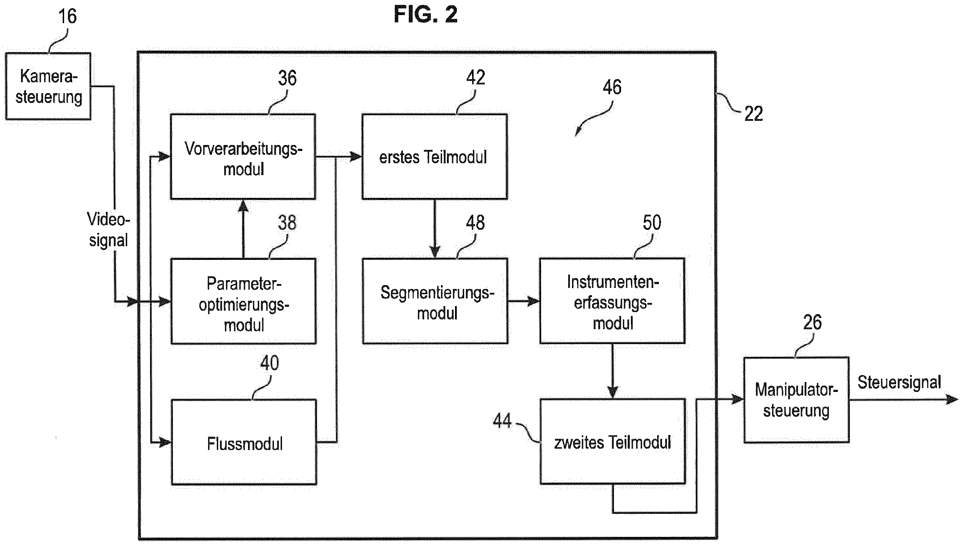

Figur 2 zeigt den Aufbau der Bildverarbeitungseinheit 22 in einem Blockdiagramm.

Die Bildverarbeitungseinheit 22 enthält ein Vorverarbeitungsmodul 36, ein Parameteroptimierungsmodul 38 und ein Flussmodul 40. Den Modulen 36, 38 und 40 wird von der Kamerasteuerung 16 jeweils das Videosignal zugeführt, das eine Bildsequenz von Einzelbildern beinhaltet. Das Parameteroptimierungsmodul 38 ist mit dem Vorverarbeitungsmodul 36 gekoppelt.

Die Bildverarbeitungseinheit 22 enthält ferner ein erstes Teilmodul 42, das zusammen mit einem zweiten Teilmodul 44 ein in Figur 2 allgemein mit 46 bezeichnetes Nachverfolgungsmodul bildet. Das erste Teilmodul 42 ist eingangsseitig mit dem Vorverarbeitungsmodul 36 und dem Flussmodul 40 gekoppelt. Ausgangsseitig ist es mit einem Segmentierungsmodul 48 verbunden. Das Segmentierungsmodul 48 ist ausgangsseitig wiederum mit einem Instrumentenerfassungsmodul 50 gekoppelt. Der Ausgang des Instrumentenmoduls 50 ist mit dem zweiten Teilmodul 44 des Nachverfolgungsmoduls 46 gekoppelt. Das zweite Teilmodul 44 bildet schließlich den Ausgang der Bildverarbeitungseinheit 22 und ist dementsprechend mit der Manipulatorsteuerung 26 verbunden, die aus dem ihr von der Bildverarbeitungseinheit 22 zugeführten Signal das Steuersignal zur Ansteuerung des Manipulators 14 generiert.

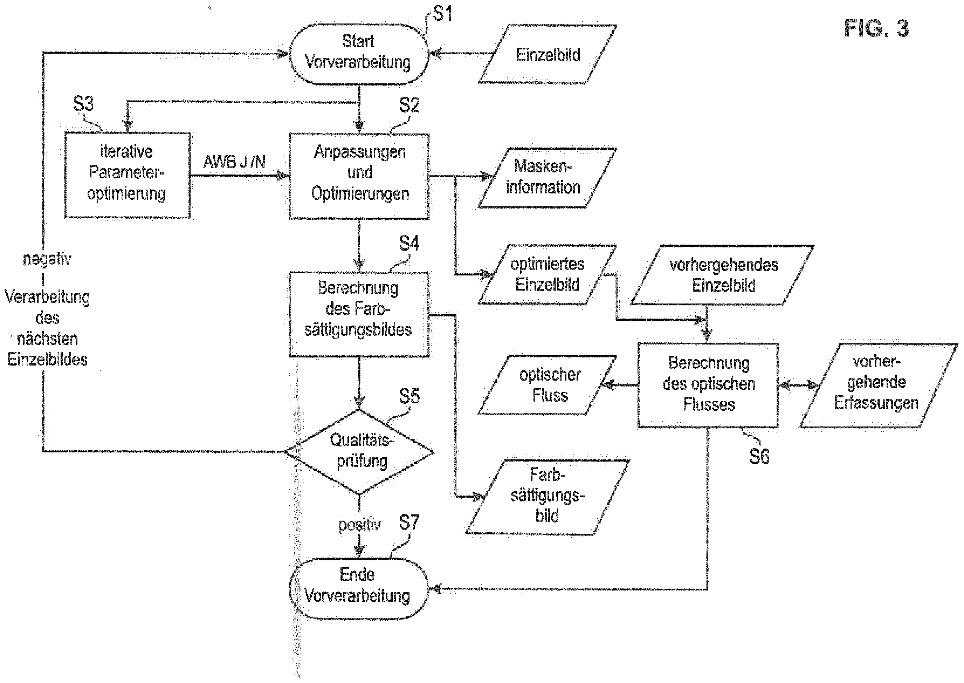

Die Funktionsweise der in der Bildverarbeitungseinheit 22 enthaltenen Module 36 bis 50 ergibt sich im Folgenden aus den in den Figuren 3, 4 und 5 dargestellten Ablaufdiagrammen, die anhand eines Beispiels die erfindungsgemäße Bildverarbeitung veranschaulichen. In den Figuren 3 bis 5 bezeichnen rechteckige Symbole die von den jeweiligen Modulen ausgeführten Verarbeitungsschritte, während rautenartige Symbole Speicher- bzw. Auslesevorgänge angeben.

Das Ablaufdiagramm nach Figur 3 zeigt eine erfindungsgemäß durchgeführte Bildvorverarbeitung, bei der insbesondere das Vorverarbeitungsmodul 36, das Parameteroptimierungsmodul 38 und das Flussmodul 40 der Bildverarbeitungseinheit 22 zum Einsatz kommen.

In Schritt S1 empfangen das Vorverarbeitungsmodul 36, das Parameteroptimierungsmodul 38 und das Flussmodul 40 ein zwischengespeichertes Einzelbild der in dem Videosignal enthaltenen Bildsequenz. In dem vorliegenden Ausführungsbeispiel wird im Folgenden davon ausgegangen, dass dieses Einzelbild ein in herkömmlicherweise erzeugtes RGB-Einzelbild ist, das häufig auch als RGB-Frame bezeichnet wird.

In Schritt S2 nimmt das Vorverarbeitungsmodul 36 an dem RGB-Einzelbild verschiedene Anpassungen und Optimierungen vor, die etwa dazu dienen, Bildgröße, Helligkeit und Kontrast geeignet einzustellen. Insbesondere führt das Vorverarbeitungsmodul 36 in Schritt S2 bei Bedarf auch einen automatischen Weißabgleich durch. Ob in S2 ein Weißabgleich erfolgt oder nicht, wird in Schritt S3 durch das Parameteroptimierungsmodul 38 über ein Ansteuersignal AWB Y/N festgelegt, welches das Parameteroptimierungsmodul 38 dem Vorverarbeitungsmodul 36 zuführt. Hierzu nimmt das Parameteroptimierungsmodul 38 an der ihm zugeführten Bildsequenz eine entsprechenden Überprüfung vor. Das Parameteroptimierungsmodul 38 arbeitet im Rahmen einer iterativen Parameteroptimierung asynchron zu dem Vorverarbeitungsmodul 36, indem es beispielsweise die vorgenannte Überprüfung nicht für jedes Einzelbild durchführt.

In Schritt S2 erfasst das Vorverarbeitungsmodul 36 zudem eine Kameramaske in dem Einzelbild, die einen nicht auswertbaren Bildbereich darstellt. Das Vorverarbeitungsmodul 36 speichert dann eine entsprechende Maskeninformation, die es im späteren Ablauf ermöglicht, den der Maske entsprechenden nicht nutzbaren Bildbereich gleichsam aus dem Einzelbild herauszufiltern.

Nachdem das Einzelbild in Schritt S2 im Hinblick auf die weitere Verarbeitung optimiert ist, wird es in einem Bildspeicher abgespeichert.

In Schritt S4 berechnet das Vorverarbeitungsmodul 36 auf Grundlage des RGB-Einzelbildes ein Grauwertbild, dessen Bildpunkten jeweils ein Grauwert zugeordnet ist, der die Farbsättigung des entsprechenden Bildpunktes des RGB-Einzelbildes repräsentiert. Somit stellt das in Schritt S4 erzeugte Grauwertbild ein Farbsättigungsbild dar. Dieses Farbsättigungsbild wird in S4 für die nächsten Verarbeitungsschritte abgespeichert.

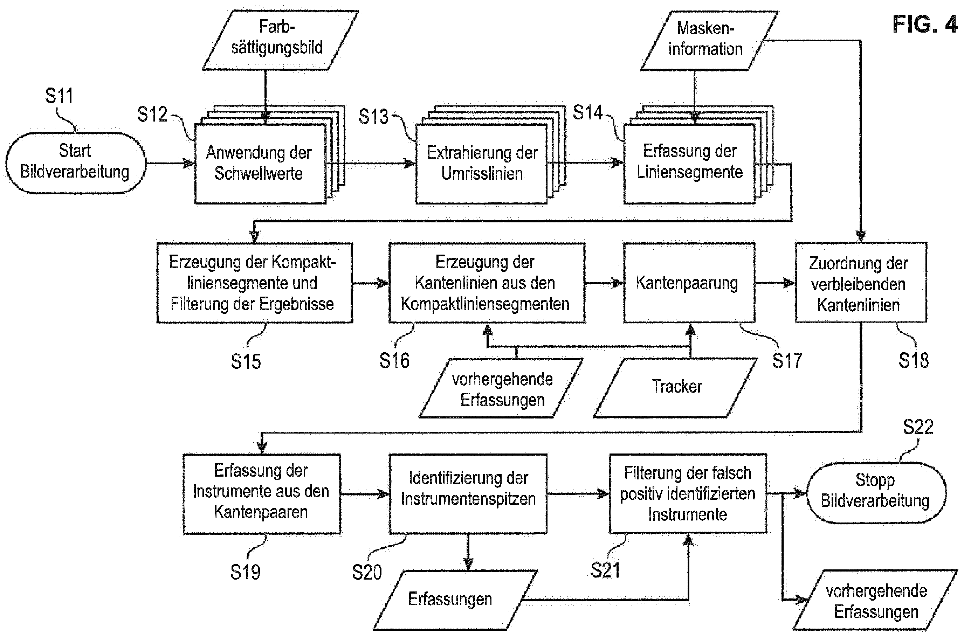

In Schritt S5 nimmt das Vorverarbeitungsmodul 36 eine Qualitätsprüfung des RGB-Einzelbildes vor. Ergibt diese Qualitätsprüfung eine negative Bewertung, so wird das RGB-Einzelbild verworfen und die Verarbeitung mit dem nächsten Einzelbild fortgesetzt. Ergibt sich dagegen in Schritt S5 eine positive Bewertung der Qualität des RGB-Einzelbildes, so endet die Vorverarbeitung, und der Prozessablauf fährt mit den Verarbeitungsschritten nach Figur 4 fort.

In Schritt S6 nach Figur 3 wird ein zu den vorstehend beschriebenen Verfahrensschritten nebenläufiger Prozess von dem Flussmodul 40 ausgeführt. Das Flussmodul 40 ist darauf ausgelegt, den optischen Fluss der in dem Videosignal enthaltenen Bildsequenz zu erfassen. Der optische Fluss repräsentiert dabei eine in der Bildsequenz enthaltene Bewegungsinformation, beispielsweise in Form eines Vektorfeldes, das Betrag und Richtung der Geschwindigkeit von Bildpunkten in der Bildsequenz angibt. Um den optischen Fluss zu erfassen, berücksichtigt das Flussmodul 40 neben dem aktuellen RGB-Einzelbild auch das vorhergehende RGB-Einzelbild. Der erfasste optische Fluss wird dann gespeichert. Außerdem erfolgt in Schritt S6 ein Abgleich mit vorhergehenden Erfassungsergebnissen.

In Schritt S7 endet die Vorverarbeitung nach Figur 3.

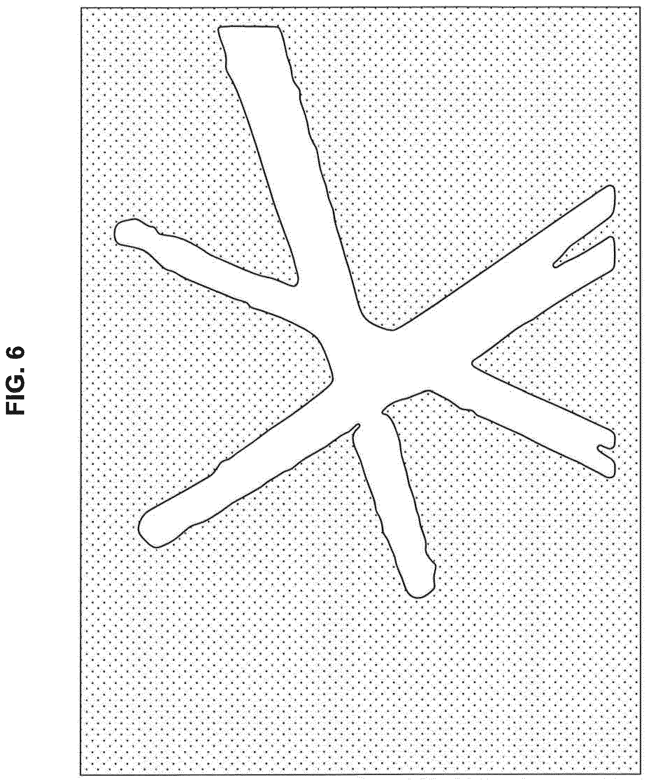

Die in Figur 4 gezeigte Bildverarbeitung wird in der Bildverarbeitungseinheit 22 vornehmlich von dem aus den beiden Teilmodulen 42, 44 gebildeten Nachverfolgungsmodul 46, dem Segmentierungsmodul 48 und dem Instrumentenerfassungsmodul 50 durchgeführt. Nach dem Start der Bildverarbeitung in Schritt S11 liest das Segmentierungsmodul 48 in Schritt S12 das Grauwertbild ein, welches das Vorverarbeitungsmodul 36 in Schritt S4 nach Figur 3 auf Grundlage des RGB-Einzelbildes berechnet und abgespeichert hat. Auf Basis dieses Grauwertbildes erzeugt das Segmentierungsmodul 48 N Binärbilder unter Anwendung unterschiedlicher Schwellwerte, die beispielsweise von dem Parameteroptimierungsmodul 38 vordefiniert werden. Diese Schwellwerte entsprechen jeweils einer vordefinierten Farbsättigung, mit der die Grauwerte der Bildpunkte des Grauwertbildes verglichen werden. Denjenigen Bildpunkten des Grauwertbildes, deren Grauwerte gleich oder kleiner als der der jeweils vordefinierten Farbsättigung entsprechende Schwellwert sind, werden in dem jeweiligen Binärbild Binärbildpunkte zugeordnet, die einen ersten Binärwert (z.B. 1) haben. Dementsprechend werden denjenigen Bildpunkten des Grauwertbildes, deren Grauwerte größer als der Schwellwert sind, in dem jeweiligen Binärbild Binärbildpunkte zugeordnet, die einen zweiten Binärwert (z.B. 0) aufweisen. Ein solches in Schritt S12 erzeugtes Binärbild ist rein beispielhaft in Figur 6 dargestellt, wobei dort Binärbildpunkten mit dem Binärwert 1 die Farbe Weiß und Binärbildpunkten mit dem Binärwert 0 die Farbe Schwarz zugeordnet ist. Die Farbe Schwarz ist in Figur 6 aus Gründen der Darstellung gepunktet gezeichnet. Somit repräsentieren in dem Binärbild nach Figur 6 weiße Bildsegmente Bildbereiche, in denen die Farbsättigung gleich oder kleiner als die jeweils vordefinierte Farbsättigung ist. Demgegenüber repräsentieren schwarze Bildsegmente Bildbereiche, in denen die Farbsättigung größer als die vordefinierte Farbsättigung ist. Da die erfindungsgemäße Bildverarbeitung von der Annahme ausgeht, das in dem Videobild zu erkennende Instrumente eine geringere Farbsättigung als die zu behandelnde anatomische Struktur aufweist, stellen in dem Binärbild nach Figur 6 die weißen Bildsegmente die zu erkennenden Instrumente dar, während die schwarzen Bildsegmente die anatomische Struktur repräsentieren.

In Schritt S12 erzeugt das Segmentierungsmodul 48 im Ergebnis N Binärbilder der in Figur 6 dargestellten Art, die sich jedoch, da ihnen N unterschiedliche Schwellwerte zugrunde liegen, mehr oder weniger voneinander unterscheiden. Je unterschiedlicher die N Schwellwerte sind, desto stärker werden sich auch die mit diesen Schwellwerten erzeugten Binärbilder voneinander unterscheiden.

In Schritt S13 extrahiert das Instrumentenerfassungsmodul 50 aus jedem der N Binärbilder Umrisslinien und speichert diese in N Umrissdatensätzen. In dem beispielhaften Binärbild nach Figur 6 verlaufend diese Umrisslinien an den Übergängen zwischen den weißen und den schwarzen Bildsegmenten.

In Schritt S14 ermittelt das Instrumentenerfassungsmodul 50 aus den N Umrissdatensätzen geradlinige Abschnitte, die im Weiteren als Liniensegmente bezeichnet werden. Die ermittelten Liniensegmente speichert das Instrumentenerfassungsmodul 50 in N Liniensegmentdatensätzen.

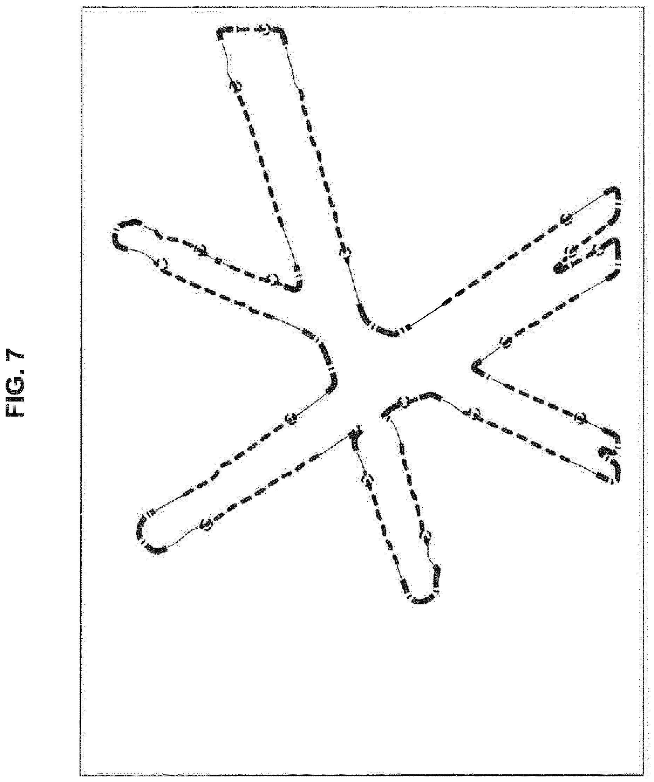

Der Prozessschritt nach Schritt S14 ist in der Darstellung nach Figur 7 veranschaulicht. Dort sind die Liniensegmente gestrichelt dargestellt.

In Schritt S14 ermittelt das Instrumentenerfassungsmodul 50 auch kurvige Abschnitte der Umrisslinien, um diese aus den weiter zu verarbeitenden Umrisslinien zu eliminieren. Diese kurvigen Abschnitte sind in Figur 7 strichpunktiert dargestellt.

Um Verfälschungen durch die Kameramaske zu vermeiden, berücksichtigt das Instrumentenerfassungsmodul 50 in Schritt S14 die Maskeninformation, welche das Vorverarbeitungsmodul 36 in Schritt S2 nach Figur 2 ermittelt und abgespeichert hat.

Ausgehend von den in Schritt S14 erzeugten N Liniensegmentdatensätzen generiert das Instrumentenerfassungsmodul 50 in Schritt S15 einen einzigen Datensatz, in dem einander entsprechende Liniensegmente der N Binärbilder jeweils zu einem einzigen Liniensegment zusammengesetzt sind. Dieses zusammengesetzte Liniensegment, im Folgenden auch als Kompaktliniensegment bezeichnet, repräsentiert eine Kantenlinie des Instrumentes, die sich gleichsam aus der Überlagerung aller N Binärbilder ergibt. Ob Liniensegmente, die in den N Binärbildern identifiziert werden, einander in vorstehendem Sinne entsprechen, beurteilt das Instrumentenerfassungsmodul 50 in Schritt S15 nach vordefinierten Kriterien.

Ein Beispiel für ein solches Kriterium ist die Parallelität der betrachteten Liniensegmente. So kann beispielsweise ein Winkel als Schwellwert vorgegeben und geprüft werden, ob die betrachteten Liniensegmente eine Winkelabweichung in ihrer Ausrichtung aufweisen, die unterhalb dieses Schwellwertes liegt. Ist dies der Fall, so wird das Kriterium der Parallelität als erfüllt angesehen. Ein weiteres Kriterium ist beispielsweise ein sogenannter Überlapp, für den wiederum ein Schwellwert vordefiniert werden kann. Weisen die betrachteten Segmente einen Überlapp miteinander auf, der diesen Schwellwert übersteigt, so wird auch dieses Kriterium als erfüllt angesehen. Ein weiteres Kriterium ist beispielsweise der Abstand zwischen den betrachteten Liniensegmenten. Ist dieser kleiner als ein vordefinierter Schwellwert, so wird dieses Kriterium als erfüllt angesehen. Auch der Grauwert repräsentiert in Kombination mit dem vorstehend genannten Segmentabstand ein geeignetes Kriterium.

Die vorstehenden Kriterien zielen im Wesentlichen darauf ab, zuverlässig festzustellen, ob die betrachteten Liniensegmente innerhalb des Grauwertbildes, welches die Grundlage für die N-Binärbilder darstellt, längs ein- und desselben Gradienten auftreten.

Das Instrumentenerfassungsmodul filtert somit in Schritt S15 die in Schritt S14 generierte Ergebnismenge auf Basis der vorstehend erläuterten Kriterien.

In Schritt S16 erstellt das Instrumentenerfassungsmodul 50 aus den Kompaktliniensegmenten, in denen einander entsprechende Liniensegmente zusammengefasst sind, Kantenlinien. Diese repräsentieren die Kanten des erfassten Instrumentes. Dabei berücksichtigt das Instrumentenerfassungsmodul 50 Erfassungsergebnisse, die aus dem vorhergehenden Einzelbild gewonnen wurden.

In Schritt S17 nimmt das Instrumentenerfassungsmodul 50 eine Paarung von jeweils zwei parallel zueinander angeordneten, in Schritt S16 ermittelten Kantenlinien vor. Diese Kantenpaarung erfolgt wiederum auf Basis vorbestimmter Kriterien. Ein solches Kriterium ist beispielsweise die Orientierung zweier Vektoren, die senkrecht zu den beiden jeweils betrachteten Kantenlinien verlaufen.

In dem Prozessschritt S17 fließen Informationen über die Positionen von Instrumenten aus dem vorhergehenden Einzelbild ein. Des Weiteren werden Positionsdaten sogenannter Tracker berücksichtigt, die jeweils als Repräsentation eines im Einzelbild erfassten Instrumentes anzusehen sind.

Üblicherweise können in Schritt S17 nicht alle Kanten mit jeweils einer anderen Kante gepaart werden. Deshalb ordnet das Instrumentenerfassungsmodul 50 in Schritt S18 übriggebliebene Kanten vermeintlichen Instrumenten zu, die sich in der Nähe des Randes des Einzelbildes befinden. Hierzu berücksichtigt das Instrumentenerfassungsmodul wiederum die in Schritt S2 nach Figur 3 bereitgestellte Maskeninformation.
In Schritt S19 ermittelt das Instrumentenerfassungsmodul 50 anhand eines jeweiligen Kantenpaars die Instrumentenachse sowie Länge und Orientierung des zugehörigen Instrumentes.

In Schritt S20 bestimmt das Instrumentenerfassungsmodul 50 die Spitze des Instrumentes unter Berücksichtigung geometrischer Eigenschaften, die das Instrumentenerfassungsmodul 50 von vorneherein als gegeben ansieht. Eine solche geometrische Eigenschaft könnte beispielsweise eine von vorneherein angenommene Konusform des Instrumentes sein. Die in Schritt S20 gewonnenen Erfassungsergebnisse werden zur weiteren Berücksichtigung in den folgenden Verarbeitungsschritten abgespeichert.

In Schritt S21 filtert das Instrumentenerfassungsmodul 50 falsch positiv ermittelte Instrumente aus. Als Kriterium hierfür kommt beispielsweise die Qualität der Kantenpaarung in Betracht. Auch können vorausgesetzte Gesetzmäßigkeiten bestimmter Farbeigenschaften als Kriterium herangezogen werden. Beispielsweise liegt eine bestimmte Farbeigenschaft entweder bei einem Instrument oder aber bei der anatomischen Struktur vor, nicht jedoch sowohl bei dem Instrument als auch bei der anatomischen Struktur. Die Berücksichtigung einer solchen Gesetzmäßigkeit ermöglicht das Ausfiltern falsch positiv erkannter Instrumente.

Mit Schritt S22 endet die Bildverarbeitung nach Figur 4.

Im Ergebnis liefert die Bildverarbeitung nach Figur 4 gespeicherte Informationen über Länge, Orientierung und Lage der Instrumentenspitze. Zudem werden ergänzende Informationen bereitgestellt, beispielsweise über die Qualität der Kantenpaarung.

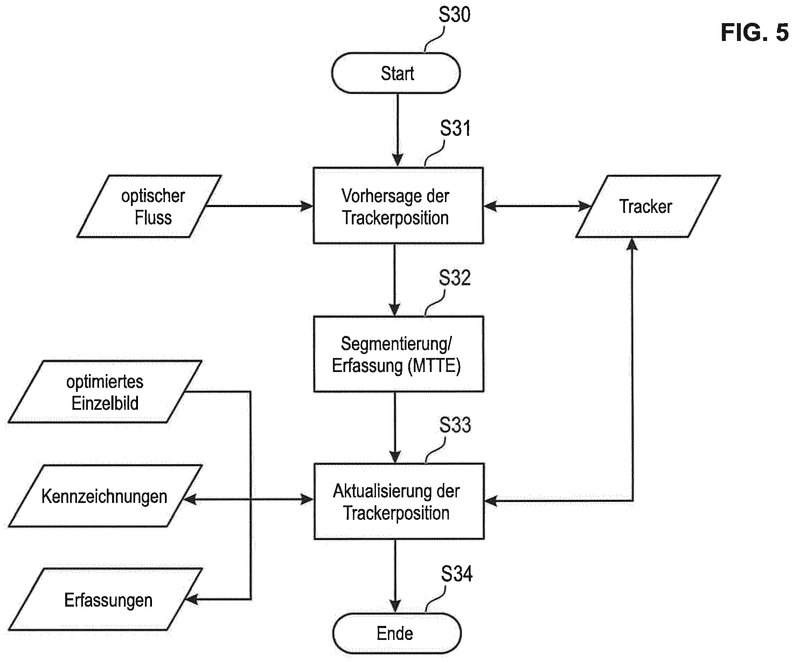

Figur 5 ist ein Ablaufdiagramm, das die Funktionsweise des aus den beiden Teilmodulen 42 und 44 gebildeten Nachverfolgungsmoduls 46 zeigt, das in der Bildverarbeitungseinheit 22 enthalten ist.

Nach dem Start in Schritt S30 korrigiert das erste Teilmodul 42 des Nachverfolgungsmoduls 46 in Schritt S31 auf Grundlage des von dem Flussmodul 40 in Schritt S6 nach Figur 3 erfassten optischen Flusses die Positionen der Tracker, die in dem letzten Einzelbild bestimmt worden sind. Dabei trifft das erste Teilmodul 42 eine Vorhersage, wohin sich die Tracker in dem vorliegenden Einzelbild bewegen werden. Diese Vorhersage der Trackerbewegung dient der Verhinderung von Lücken und Sprüngen.

In Schritt S32 führen dann das Segmentierungsmodul 48 und das Instrumentenerfassungsmodul 50 die in Figur 4 dargestellten Verarbeitungsschritte für das vorliegende Einzelbild aus.

In Schritt S33 aktualisiert das zweite Teilmodul 44 des Nachverfolgungsmoduls 46 auf Grundlage der in Schritt S20 nach Figur 4 gewonnenen Erfassungsergebnisse die Positionen der Tracker und generiert bei Bedarf neue Tracker, wenn in dem Einzelbild neue Instrumente erfasst werden. Das zweite Teilmodul 44 überprüft dabei die von dem ersten Teilmodul 42 in Schritt S31 getroffene Vorhersage der Trackerpositionen. Anhand bestimmter Kriterien ermittelt das zweite Teilmodul 44 die Vertrauenswürdigkeit der aktuell verwendeten Tracker.

Das zweite Teilmodul 44 ordnet in Schritt S33 den Trackern Kennzeichnungen (Label) zu, die auf dem Sichtgerät 18 beispielsweise in unterschiedlichen Farben dargestellt werden. Eine solche Kennzeichnung verschiedener Tracker ist beispielsweise dann von Vorteil, wenn ein Instrument zunächst mit einem bestimmten Tracker A nachverfolgt wird, dann aus der Nachverfolgung herausfällt, so dass der Tracker A gelöscht wird, und schließlich dasselbe Instrument wieder in die Nachverfolgung aufgenommen wird, so dass hierfür ein neuer Tracker B erzeugt wird. In diesem Fall wird den beiden Trackern A und B ein- und dieselbe Kennzeichnung zugeordnet.

Das zweite Teilmodul 44 speichert dann in Schritt S33 die Erfassungsergebnisse für den nächsten Durchlauf.

In Schritt S34 endet der Prozess nach Figur 5.

Die vorstehend unter Bezugnahme auf die Figuren 3 bis 5 erläuterte Bildverarbeitung ist lediglich beispielhaft zu verstehen. So ist es nicht unbedingt erforderlich, auf Grundlage des jeweiligen Grauwertbildes eine Vielzahl von Binärbildern unter Anwendung unterschiedlicher Schwellwerte zu generieren. Häufig reicht die Erzeugung eines einzigen Binärbildes mit Hilfe eines geeignet gewählten Schwellwertes aus, um Bildsegmente geringer Farbsättigung, die in dem Einzelbild ein Instrument repräsentieren, von Bildsegmenten hoher Farbsättigung zu separieren, welche die anatomische Struktur darstellen.

Dies gilt beispielsweise in Fällen, in denen nur Instrumente gleichen Materials und damit gleicher Farbsättigung zur Anwendung kommen.

### Bezugszeichenliste

- 10: Assistenzeinrichtung
- 12: Kamera
- 14: Manipulator
- 16: Kamerasteuerung
- 18: Sichtgerät
- 20: Steuerung
- 22: Bildverarbeitungseinheit
- 24: Steuereinheit
- 26: Manipulatorsteuerung
- 28: Betätigungsschalter
- 30: Schnittstellensteuereinheit
- 32: grafische Benutzerschnittstelle
- 34: Eingabegeräte
- 36: Vorverarbeitungsmodul
- 38: Parameteroptimierungsmodul
- 40: Flussmodul
- 42: erstes Teilmodul
- 44: zweites Teilmodul
- 46: Nachverfolgungsmodul
- 48: Segmentierungsmodul
- 50: Instrumentenerfassungsmodul

## Patentansprüche

1. Assistenzeinrichtung (10) zur bildgebenden Unterstützung eines Operateurs während eines chirurgischen Eingriffs unter Verwendung mindestens eines medizinischen Instrumentes, mit
einer Kamera (12) zum Erzeugen eines Videosignals, das eine Bildsequenz von Einzelbildern beinhaltet;
einem Sichtgerät (18) zum Darstellen der Bildsequenz auf Grundlage des Videosignals;
einer Bildverarbeitungseinheit (22) mit einem Instrumentenerfassungsmodul (50) zum Erfassen mindestens einer das verwendete Instrument in dem jeweiligen Einzelbild repräsentierenden Zielstruktur durch Identifizieren eines vorbestimmten Erkennungsmerkmals und zum Extrahieren einer Positionsinformation, welche die Lage der Zielstruktur in dem Einzelbild angibt;
einem mit der Kamera (12) gekoppelten Manipulator (14), der zum Bewegen der Kamera (12) über ein Steuersignal ansteuerbar ist; und
einer Manipulatorsteuerung (26) zum Erzeugen des Steuersignals aus der Positionsinformation und zum Ansteuern des Manipulators (14) über das Steuersignal;
**dadurch gekennzeichnet, dass** das Instrumentenerfassungsmodul (50) als vordefiniertes Erkennungsmerkmal ein Bildsegment identifiziert, das durch eine Farbsättigung, die gleich oder kleiner als eine vordefinierte Farbsättigung ist, und
eine das Bildsegment begrenzende Umrisslinie charakterisiert ist, die mindestens einen geradlinigen Abschnitt aufweist,
dass die Bildverarbeitungseinheit (22) ein Vorverarbeitungsmodul (36) enthält, das auf Grundlage des jeweiligen Einzelbildes ein Grauwertbild erzeugt, dessen Bildpunkten jeweils ein Grauwert zugeordnet ist, der die Farbsättigung des entsprechenden Bildpunktes des Einzelbildes repräsentiert;
dass die Bildverarbeitungseinheit (22) ein Segmentierungsmodul (48) enthält, das auf Grundlage des jeweiligen Grauwertbildes mehrere Binärbilder erzeugt und für deren Erzeugung unterschiedliche Schwellwerte anwendet, in denen das Instrumentenerfassungsmodul (50) das Bildsegment identifiziert,
und dass das Instrumentenerfassungsmodul (50) in den erzeugten Binärbildern einander entsprechende geradlinige Abschnitte identifiziert und diese zu einer einzigen Kantenlinie zusammenfasst, die eine Kante des Instrumentes repräsentiert.

2. Assistenzeinrichtung (10) nach Anspruch 1, **dadurch gekennzeichnet, dass**
den Binärbildpunkten eines jeden Binärbilds ein erster Binärwert zugeordnet ist, wenn die zugehörigen Grauwerte gleich oder kleiner als ein der vordefinierten Farbsättigung entsprechender Schwellwert ist, und den Binärbildpunkten ein zweiter Binärwert zugeordnet ist, wenn die zugehörigen Grauwerte größer als der Schwellwert sind.

3. Assistenzeinrichtung (10) nach Anspruch 1 oder 2, **dadurch gekennzeichnet, dass** das Instrumentenerfassungsmodul (50) mehrere kollinear voneinander beabstandete geradlinige Abschnitte der Umrisslinie zu einer durchgehenden Kantenlinie zusammensetzt, die eine Kante des Instrumentes repräsentiert.

4. Assistenzeinrichtung (10) nach Anspruch 3, **dadurch gekennzeichnet, dass** das Instrumentenerfassungsmodul (50) zur Erfassung der Zielstruktur jeweils zwei parallel zueinander angeordnete Kantenlinien miteinander paart.

5. Assistenzeinrichtung (10) nach Anspruch 4, **dadurch gekennzeichnet, dass** das Instrumentenerfassungsmodul (50) auf Grundlage der beiden miteinander gepaarten Kantenlinien eine Instrumentenspitze identifiziert.

6. Assistenzeinrichtung (10) nach einem der vorhergehenden Ansprüche, **dadurch gekennzeichnet, dass**
die Bildverarbeitungseinheit (22) ein Nachverfolgungsmodul (46) enthält, das die von dem Instrumentenerfassungsmodul (50) erfasste Zielstruktur über mehrere aufeinander folgende Einzelbilder nachverfolgt; und
die Manipulatorsteuerung (26) das auf das jeweilige Einzelbild bezogene Steuersignal zur Ansteuerung des Manipulators (14) aus der Positionsinformation der Zielstruktur nur dann erzeugt, wenn diese Zielstruktur von dem Nachverfolgungsmodul (46) schon über mehrere aufeinander folgende Einzelbilder nachverfolgt worden ist.

7. Assistenzeinrichtung (10) nach Anspruch 6, **dadurch gekennzeichnet, dass** das Nachverfolgungsmodul (46) der von dem Instrumentenerfassungsmodul (50) in dem jeweiligen Einzelbild erstmalig erfassten Zielstruktur einen Tracker zuordnet und diese in den nachfolgenden Einzelbildern erfasste Zielstruktur anhand des Trackers nachverfolgt.

8. Assistenzeinrichtung (10) nach einem der vorhergehenden Ansprüche, **dadurch gekennzeichnet, dass** die Bildverarbeitungseinheit (22) ein Flussmodul (40) enthält, das einen optischen Fluss der Bildsequenz erfasst, der eine in der Bildsequenz enthaltene Bewegungsinformation repräsentiert.

9. Assistenzeinrichtung (10) nach einem der Ansprüche 6 bis 8, **dadurch gekennzeichnet, dass**
das Nachverfolgungsmodul (46) ein dem Instrumentenerfassungsmodul (50) vorgeordnetes erstes Teilmodul (42) und ein dem Instrumentenerfassungsmodul (50) nachgeordnetes zweites Teilmodul (44) umfasst;
das erste Teilmodul (42) unter Berücksichtigung des von dem Flussmodul (40) erfassten optischen Flusses für das nächste Einzelbild, das von dem Instrumentenerfassungsmodul (50) noch nicht verarbeitet worden ist, eine Vorhersage über die Positionsinformation des Trackers trifft; und
das zweite Teilmodul (40) für dieses nächste Einzelbild, das von dem Instrumentenerfassungsmodul (50) verarbeitet worden ist, die von dem ersten Teilmodul (42) getroffene Vorhersage anhand der von dem Instrumentenerfassungsmodul (50) erfassten Positionsinformation des Trackers überprüft.

10. Assistenzeinrichtung (10) nach einem der Ansprüche 2 bis 9, **dadurch gekennzeichnet, dass** das Vorverarbeitungsmodul (36) ausgebildet ist, an dem jeweiligen Einzelbild einen Weißabgleich vorzunehmen.

11. Assistenzeinrichtung (10) nach Anspruch 10, **dadurch gekennzeichnet, dass** die Bildverarbeitungseinheit (22) ein Parameteroptimierungsmodul (38) enthält, das die Einzelbilder asynchron zu dem Vorverarbeitungsmodul (36) verarbeitet und daraus eine Ansteuerinformation generiert, die dem Vorverarbeitungsmodul (36) vorgibt, ob es den Weißabgleich vorzunehmen hat oder nicht.

12. Assistenzeinrichtung (10) nach Anspruch 11, **dadurch gekennzeichnet, dass** das Parameteroptimierungsmodul (38) auf Grundlage der asynchronen Verarbeitung der Einzelbilder die Schwellwerte für das Segmentierungsmodul (48) vordefiniert.

13. Verfahren zur bildgebenden Unterstützung eines Operateurs während eines chirurgischen Eingriffs unter Verwendung mindestens eines medizinischen Instrumentes, bei dem
mittels einer Kamera (12) ein Videosignal erzeugt wird, das eine Bildsequenz von Einzelbildern beinhaltet;
die Bildsequenz auf Grundlage des Videosignals auf einem Sichtgerät (18) dargestellt wird;
mindestens eine das verwendete Instrument in dem jeweiligen Einzelbild repräsentierende Zielstruktur durch Identifizieren eines vordefinierten Erkennungsmerkmals erfasst und eine Positionsinformation, welche die Lage der Zielstruktur in dem Einzelbild angibt, extrahiert wird; und
ein Steuersignal aus der Positionsinformation erzeugt und ein mit der Kamera (12) gekoppelter Manipulator (14) über das Steuersignal angesteuert wird, um die Kamera (12) zu bewegen;
**dadurch gekennzeichnet, dass** als vordefiniertes Erkennungsmerkmal ein Bildsegment identifiziert wird, das durch eine Farbsättigung, die gleich oder kleiner als eine vordefinierte Farbsättigung ist, und eine das Bildsegment begrenzende Umrisslinie charakterisiert ist, die mindestens einen geradlinigen Abschnitt aufweist,
dass auf Grundlage des jeweiligen Einzelbildes ein Grauwertbild erzeugt wird, dessen Bildpunkten jeweils ein Grauwert zugeordnet ist, der die Farbsättigung des entsprechenden Bildpunktes des Einzelbildes repräsentiert;
dass auf Grundlage des jeweiligen Grauwertbildes mehrere Binärbilder erzeugt und für deren Erzeugung unterschiedliche Schwellwerte angewendet werden, in denen das Bildsegment identifiziert wird,
und dass in den erzeugten Binärbildern einander entsprechende geradlinige Abschnitte identifiziert werden und diese zu einer einzigen Kantenlinie zusammengefasst werden, die eine Kante des Instrumentes repräsentiert.

## Claims

1. An assistance device (10) for imaging support for a surgeon during a surgical procedure using at least one medical instrument, comprising
a camera (12) for generating a video signal that includes an image sequence of individual images;
a display device (18) for displaying the image sequence based on the video signal;
an image processing unit (22) having an instrument detection module (50) for detecting at least one target structure representing the instrument used in the respective individual image, and doing so by identifying a predefined distinguishing feature, and for extracting position information indicating the position of the target structure in the individual image;
a manipulator (14), which is coupled to the camera (12) and can be triggered for moving said camera (12) by means of a control signal; and
a manipulation controller (26) for generating the control signal from the position information and for triggering the manipulator (14) by means of the control signal;
**characterized in that** the instrument detection module (50) identifies as a predefined distinguishing feature an image segment that is **characterized by** a color saturation, which is the same as or less than a predefined color saturation, and by an outline delimiting the image segment, said outline having at least one rectilinear segment;
in that the image processing unit (22) includes a preprocessing module (36), which generates a gray-value image on the basis of the respective individual image, each pixel of the gray-value image being assigned a gray value representing the color saturation of the corresponding pixel of the individual image;
in that the image processing unit (22) includes a segmentation module (48), which generates a plurality of binary images on the basis of the respective gray-value image and uses for generating these images different threshold values in which the instrument detection module (50) identifies the image segment;
and in that the instrument detection module (50) identifies mutually corresponding rectilinear segments in the binary images thereby generated and combines them into a single edge line representing one edge of the instrument.

2. The assistance device (10) according to claim 1, **characterized in that** a first binary value is assigned to the binary image products of each binary image if the respective gray values are equal to or less than a threshold value corresponding to the predefined color saturation, and a second binary value is assigned to the binary pixels if the respective gray values are greater than the threshold value.

3. The assistance device (10) according to claim 1 or 2, **characterized in that** the instrument detection module (50) combines a plurality of rectilinear segments of the delineating line, such that the segments are mutually colinear by a distance to form a continuous edge line representing one edge of the instrument.

4. The assistance device (10) according to claim 3, **characterized in that** the instrument detection module (50) pairs two edge lines, which are arranged in mutual parallel, with one another to detect the target structure.

5. The assistance device (10) according to claim 4, **characterized in that** the instrument detection module (50) identifies an instrument tip on the basis of the two edge lines paired with one another.

6. The assistance device (10) according to any one of the preceding claims, **characterized in that**
the image processing unit (22) includes a tracking module (46), which tracks the target structure detected by the instrument detection module (50) over a plurality of successive individual images; and
the manipulator controller (26) then generates the control signal, based on the respective individual image, for triggering the manipulator (14) from the position information of the target structure only if this target structure has already been tracked by the tracking module (46) over a plurality of successive individual images.

7. The assistance device (10) according to claim 6, **characterized in that** the tracking module (46) assigns a tracker to the target structure, which is detected for the first time by the instrument detection module (50) in the respective individual image, and the tracking module tracks this target structure detected in the subsequent individual images on the basis of the tracker.

8. The assistance device (10) according to any one of the preceding claims, **characterized in that** the image processing unit (22) contains a flow module (40), which detects an optical flow of the image sequence representing motion information contained in the image sequence.

9. The assistance device (10) according to any one of claims 6 to 8, **characterized in that**
the tracking module (46) comprises a first submodule (42) upstream from the instrument detection module (50) and a second submodule (44) downstream from the instrument detection module (50);
the first submodule (42) makes a prediction about the position information of the tracker, taking into account the optical flow detected by the flow module (40) for the next individual image, which has not yet been processed by the instrument detection module (50); and
the second submodule (40) checks the prediction made by the first submodule (42) for this next individual image, which has been processed by the instrument detection module (50) on the basis of the position information of the provided by the tracker and detected by the instrument detection module (50).

10. The assistance device (10) according to any one of claims 2 to 9, **characterized in that** the preprocessing module (36) is designed to perform a whiteness comparison on the respective individual image.

11. The assistance device (10) according to claim 10, **characterized in that** the image processing unit (22) includes a parameter optimization module (38), which processes the individual images asynchronously with the preprocessing module (36) and generates therefrom control information indicating to the preprocessing module (36) whether or not it has performed the whiteness comparison.

12. The assistance device (10) according to claim 11, **characterized in that** the parameter optimization module (38) predefines the threshold values for the segmentation module (48) on the basis of the asynchronous processing of the individual images.

13. A method for imaging support for a surgeon during a surgical procedure using at least one medical instrument, wherein
by means of a camera (12) a video signal is generated, including an image sequence of individual images;
the image sequence is displayed on a display device (18) based on the video signal;
at least one target structure representing the instrument used in the respective individual image is detected by identifying a predefined distinguishing feature, and position information indicating the location of the target structure in the individual image is extracted; and
a control signal is generated from the position information, and a manipulator (14) coupled to the camera (12) is triggered by the control signal to move the camera (12);
**characterized in that** an image segment, which is **characterized by** a color saturation that is the same as or less than a predefined color saturation and by an outline delineating the image segment, comprising at least one rectilinear segment, is identified as the predefined distinguishing feature,
in that a gray-value image is generated on the basis of the respective individual image, each pixel being assigned a gray value representing the color saturation of the corresponding pixel of the individual image;
in that a plurality of binary images is generated on the basis of the respective gray-value image, and various threshold values, in which the image segment is identified, are used to generate the binary images,
and in that mutually corresponding rectilinear sections are identified in the binary images thereby generated, and these rectilinear sections are combined to form a single edge line representing one edge of the instrument.

## Revendications

1. Dispositif d'assistance (10) permettant d'assister un opérateur par imagerie pendant une intervention chirurgicale à l'aide d'au moins un instrument médical, le dispositif d'assistance comprenant
une caméra (12) destinée à générer un signal vidéo qui contient une séquence d'images individuelles ;
une unité de visualisation (18) destinée à présenter la séquence d'images sur la base du signal vidéo ;
une unité de traitement d'image (22) comprenant un module de détection d'instrument (50) destiné à détecter au moins une structure cible, représentant l'instrument utilisé dans l'image individuelle respective, par identification d'une caractéristique de reconnaissance prédéterminée et extraction d'une information de position qui indique l'emplacement de la structure cible dans l'image individuelle ;
un manipulateur (14) couplé à la caméra (12) et pouvant être commandé par le biais d'un signal de commande pour déplacer la caméra (12) ; et
une commande de manipulateur (26) destinée à générer le signal de commande à partir de l'information de position et à entraîner le manipulateur (14) par le biais du signal de commande ;
**caractérisé en ce que**
le module de détection d'instrument (50) identifie comme caractéristique de reconnaissance prédéfinie un segment d'image qui est **caractérisé par** une saturation de couleur égale ou inférieure à une saturation de couleur prédéfinie, et par un contour qui délimite le segment d'image et qui comporte au moins une partie rectiligne,
l'unité de traitement d'image (22) contient un module de prétraitement (36) qui génère sur la base de l'image individuelle respective une image à valeurs de gris dont chaque pixel est associé à une valeur de gris qui représente la saturation de couleur du pixel correspondant de l'image individuelle ;
l'unité de traitement d'image (22) contient un module de segmentation (48) qui génère une pluralité d'images binaires sur la base de l'image à valeurs de gris respective et qui utilise, pour générer lesdites images binaires, différentes valeurs de seuil dans lesquelles le module de détection d'instrument (50) identifie le segment d'image,
et le module de détection d'instrument (50) identifie des parties rectilignes correspondantes dans les images binaires générées et les combine pour obtenir une seule ligne de bord qui représente un bord de l'instrument.

2. Dispositif d'assistance (10) selon la revendication 1, **caractérisé en ce que**
une première valeur binaire est associée aux pixels binaires de chaque image binaire si les valeurs de gris associées sont égales ou inférieures à une valeur de seuil correspondant à la saturation de couleur prédéfinie, et une deuxième valeur binaire est associée aux pixels binaires si les valeurs de gris associées sont supérieures à la valeur de seuil.

3. Dispositif d'assistance (10) selon la revendication 1 ou 2, **caractérisé en ce que** le module de détection d'instrument (50) compose une pluralité de parties rectilignes, espacées de manière colinéaire les unes des autres, du contour pour obtenir une ligne de bord continu qui représente un bord de l'instrument.

4. Dispositif d'assistance (10) selon la revendication 3, **caractérisé en ce que** le module de détection d'instrument (50) apparie à chaque fois deux lignes de bord parallèles entre elles pour détecter la structure cible.

5. Dispositif d'assistance (10) selon la revendication 4, **caractérisé en ce que** le module de détection d'instrument (50) identifie une pointe d'instrument sur la base des deux lignes de bord appariées.

6. Dispositif d'assistance (10) selon l'une des revendications précédentes, **caractérisé en ce que**
l'unité de traitement d'image (22) contient un module de suivi (46) qui suit la structure cible, détectée par le module de détection d'instrument (50), sur une pluralité d'images individuelles successives ; et
la commande de manipulateur (26) génère le signal de commande, relatif à l'image individuelle respective, pour commander le manipulateur (14) à partir de l'information de position de la structure cible uniquement si cette structure cible a déjà été suivie par le module de suivi (46) sur une pluralité d'images individuelles successives.

7. Dispositif d'assistance (10) selon la revendication 6, **caractérisé en ce que** le module de suivi (46) associe un suiveur à la structure cible détectée pour la première fois par le module de détection d'instrument (50) dans l'image individuelle respective et suit cette structure cible, détectée dans les images individuelles suivantes, à l'aide du suiveur.

8. Dispositif d'assistance (10) selon l'une des revendications précédentes, **caractérisé en ce que** l'unité de traitement d'images (22) contient un module de flux (40) qui détecte un flux optique de la séquence d'images, lequel représente une information de mouvement contenue dans la séquence d'images.

9. Dispositif d'assistance (10) selon l'une des revendications 6 à 8, **caractérisé en ce que**
le module de suivi (46) comprend un premier sous-module (42) disposé en amont du module de détection d'instrument (50) et un deuxième sous-module (44) disposé en aval du module de détection d'instrument (50) ;
le premier sous-module (42), avec prise en compte du flux optique détecté par le module de flux (40) et relatif à l'image individuelle suivante qui n'a pas encore été traitée par le module de détection d'instrument (50), effectue une prédiction concernant l'information de position du suiveur ; et
le deuxième sous-module (40) vérifie, pour cette image individuelle suivante qui a été traitée par le module de détection d'instrument (50), la prédiction effectuée par le premier sous-module (42) sur la base de l'information de position du suivi détectée par le module de détection d'instrument (50).

10. Dispositif d'assistance (10) selon l'une des revendications 2 à 9, **caractérisé en ce que** le module de prétraitement (36) est conçu pour effectuer un équilibrage des blancs au niveau de l'image individuelle respective.

11. Dispositif d'assistance (10) selon la revendication 10, **caractérisé en ce que** l'unité de traitement d'image (22) contient un module d'optimisation de paramètres (38) qui traite les images individuelles de manière asynchrone par rapport au module de prétraitement (36) et génère à partir de là une information de commande qui prescrit au module de prétraitement (36) d'effectuer ou non un équilibrage des blancs.

12. Dispositif d'assistance (10) selon la revendication 11, **caractérisé en ce que** le module d'optimisation de paramètres (38) prédéfinit les valeurs de seuil relatives au module de segmentation (48) sur la base du traitement asynchrone des images individuelles.

13. Procédé d'assistance permettant d'assister un opérateur par imagerie pendant une intervention chirurgicale à l'aide d'au moins un instrument médical, dans lequel
un signal vidéo, qui contient une séquence d'images individuelles, est généré au moyen d'une caméra (12) ;
la séquence d'images est présentée sur une unité de visualisation (18) sur la base du signal vidéo ;
au moins une structure cible, représentant dans l'image individuelle respective l'instrument utilisé, est détectée par identification d'une caractéristique de détection prédéfinie et une information de position est extraite qui indique l'emplacement de la structure cible dans l'image individuelle ; et
un signal de commande est généré à partir de l'information de position et un manipulateur (14) couplé à la caméra (12) est commandé par le biais du signal de commande pour déplacer la caméra (12) ;
**caractérisé en ce que**
un segment d'image est identifié comme caractéristique d'identification prédéfinie, lequel est **caractérisé par** une saturation de couleur égale ou inférieure à une saturation de couleur prédéfinie et par un contour qui délimite le segment d'image et qui comporte au moins une partie rectiligne,
une image à valeurs de gris est générée sur la base de l'image individuelle respective, chaque pixel de l'image à valeurs de gris étant associé à une valeur de gris représentant la saturation de couleur du pixel correspondant de l'image individuelle ;
une pluralité d'images binaires sont générées sur la base de l'image à valeurs de gris respective et, pour générer lesdites images binaires, différentes valeurs de seuil sont utilisées dans lesquelles le segment d'image est identifié,
et des parties rectilignes correspondantes sont identifiée dans les images binaires générées et sont combinées pour obtenir une seule ligne de bord représentant un bord de l'instrument.
